# EUROPÄISCHE PATENTANMELDUNG

(11) **EP 3 939 489 A1**
(43) Veröffentlichungstag der Anmeldung: **19.01.2022**
(21) Anmeldenummer: 21184567.2
(22) Anmeldetag: 08.07.2021
(51) Int. Cl.: A61B 1/00, B21D 26/033

(54) **VERFAHREN ZUR HERSTELLUNG EINES CHIRURGISCHEN HANDGERÄTS UND EIN CHIRURGISCHES HANDGERÄT**

(30) Priorität: 14.07.2020 DE 102020118536
(71) Anmelder: Olympus Winter & Ibe GmbH, 22045 Hamburg (DE)
(72) Erfinder: Brockmann, Christian, 21279 Hollenstedt (DE); Offt, Andreas, 21465 Reinbek (DE)
(74) Vertreter: Hoener, Matthias

(57) **Zusammenfassung**

Chirurgische Handgeräte sind im Wesentlichen aus einem Geräte- bzw. Hauptkörper und einem rohrartigen Schaft aufgebaut. Bei der Herstellung bekannter chirurgischer Geräte werden die rohrartigen Schäfte mit den Gerätekörpern verschweißt. Dazu ist eine Vielzahl von sehr aufwendigen Arbeitsschritten notwendig. Aufgrund der hohen Qualitätsanforderungen an chirurgische Handgeräte hinsichtlich mechanischer Stabilität sowie Sterilität, muss die Schweißnaht höchsten Anforderungen genügen. Die Erfindung schafft ein Verfahren zur Herstellung eines chirurgischen Handgeräts, welches besonders einfach und zuverlässig anwendbar ist. Das wird dadurch erreicht, dass mindestens ein Gerätekörper (18) sowie mindestens ein rohrartiger Schaft (13) eines chirurgischen Handgerätes durch Innen-Hochdruck-Umformung miteinander formschlüssig verbunden werden.

## Beschreibung

Die Erfindung betrifft ein Verfahren zur Herstellung eines chirurgischen Handgeräts gemäß dem Oberbegriff des Anspruchs 1. Des Weiteren betrifft die Erfindung ein chirurgisches Handgerät gemäß dem Anspruch 8.

Chirurgische Handgeräte, wie beispielsweise Endoskope, Resektoskope, Zystoskope und dergleichen, sind im Wesentlichen aus einem Geräte- bzw. Hauptkörper und einem rohrartigen Schaft aufgebaut. Bei der Herstellung bekannter chirurgischer Geräte werden die rohrartigen Schäfte mit den Gerätekörpern verschweißt. Dazu ist eine Vielzahl von teilweise sehr aufwendigen und zeitintensiven Arbeitsschritten notwendig. So muss zum Beispiel zunächst der rohrartige Schaft aufgedornt werden und sodann das aufgedornte Ende des Schafts in den Gerätekörper bzw. in einen Konus des Gerätekörpers eingepresst werden. Schließlich werden die beiden Bauteile des chirurgischen Handgerätes miteinander verschweißt. Aufgrund der hohen Qualitätsanforderungen an chirurgische Handgeräte hinsichtlich mechanischer Stabilität sowie Sterilität, muss die Schweißnaht höchsten Anforderungen genügen. Diese Anforderungen sind nur mit einem großen Aufwand zu erreichen, wobei letztendlich nie sichergestellt werden kann, ob die Schweißnaht auch nach mehrmaliger Anwendung des Geräts noch den Anforderungen genügt.

Der vorliegenden Erfindung liegt die Aufgabe zugrunde, ein Verfahren zur Herstellung eines chirurgischen Handgeräts und ein chirurgisches Handgerät zu schaffen, welches eine besonders einfache und zuverlässige Herstellung zulässt.

Eine Lösung dieser Aufgabe wird durch die Maßnahmen des Anspruchs 1 beschrieben. Demnach ist es vorgesehen, dass mindestens ein Gerätekörper sowie mindestens ein rohrartiger Schaft eines chirurgischen Handgerätes, insbesondere eines Endoskops, eines Resektoskops, eines Zystoskops oder dergleichen, durch Innen-Hochdruck-Umformung (IHU) miteinander formschlüssig verbunden werden. Durch dieses IHU-Verfahren kann auf eine sehr einfache und zuverlässige Art und Weise der mindestens eine Schaft mit dem mindestens einen Gerätekörper formschlüssig verbunden werden. Durch die Einfachheit sowie die hohe Reproduzierbarkeit dieser Verbindung kann nicht nur ein qualitativ hochwertiges Gerät hergestellt, sondern auch die Produktionskosten reduziert werden.

Insbesondere sieht es die Erfindung vor, dass es sich bei dem mindestens einem Gerätekörper um einen Hauptkörper, einen Anschlusskörper oder einen Konus des Gerätekörpers handelt. Gleichermaßen ist es jedoch auch denkbar, dass andere Körper chirurgischer Geräte, wie beispielsweise eine Optikplatte, mit dem mindestens einen rohrartigen Schaft erfindungsgemäß verbunden werden.

Weiter sieht es die Erfindung bevorzugt vor, dass es sich bei dem mindestens einen rohrartigen Schaft um einen Schaft, ein Rohr oder ein Außen- bzw. Innenrohr handelt. Darüber hinaus ist es denkbar, dass weitere schaftartige bzw. rohrartige Komponenten eines chirurgischen Handgeräts auf die erfindungsgemäße Art und Weise mit dem vorgenannten Körper formschlüssig verbunden werden. Durch das hier beschriebene Verfahren lassen sich somit sämtliche Verbindungen zwischen Körpern und rohrartigen Schäften auf eine einfache und zuverlässige Art und Weise miteinander verbinden.

Das erfindungsgemäße Verfahren schließt auch Verbindungen zwischen mehreren Körpern mit mehreren Schäften ein. So kann beispielsweise ein durchgehender rohrartiger Schaft mit einem veränderlichen Querschnitt als Schaftrohr (distal im Einführbereich) und Optikführungsrohr (proximal zwischen Hauptkörper und Optikplatte) ausgebildet sein. Auf diesen Schaft kann sodann der Hauptkörper mittig und die Optikplatte am Ende platziert werden. Durch das IHU-Verfahren können dann beide Körper zeitgleich oder nacheinander auf dem Schaft fixiert bzw. mit dem Schaft verbunden werden. Gleichermaßen ist es denkbar, dass zwei Schäfte in einem Körper oder zwei Körper mit zwei Schäften, etc. miteinander verbunden werden. Gleichermaßen ist es auch denkbar, dass durch das erfindungsgemäße Verfahren mehrere Schäfte formschlüssig miteinander verbunden werden.

Bevorzugt sieht es das erfindungsgemäße Verfahren vor, dass zunächst ein Abschnitt, vorzugsweise ein Endabschnitt oder ein mittlerer Abschnitt, des mindestens einen rohrartigen Schaftes in eine Öffnung bzw. einen Aufnahmeraum des mindestens einen Gerätekörpers geführt wird. Je nach Art sowie Form und Ausgestaltung des Gerätes bzw. des Gerätekörpers wird der Schaft entsprechend weit in diesen Raum geführt. Dabei kann es sich sowohl um einen geraden als auch um einen, insbesondere beliebig, gebogenen Abschnitt des Schaftes handeln. Im Weiteren werden der mindestens eine Gerätekörper und der mindestens eine rohrartige Schaft wenigstens bereichsweise in mindestens einer Form, die vorzugsweise aus zwei Formhälften zusammengesetzt wird, fixiert. Diese Ausrichtung sowie Fixierung der Komponenten in der Form bzw. den Formteilen kann manuell oder maschinell bzw. halbautomatisch oder vollautomatisch erfolgen. Darüber hinaus ist es auch denkbar, dass die Form aus mehreren Formteilen zusammengesetzt wird. Die Form bzw. die Formteile sind genau so ausgestaltet, dass sie ein Komplementär bzw. ein Negativ der Formen des Gerätekörpers und des Schaftes bilden. Durch diese Fixierung der Komponenten in der Form wird während des Verfahrens sichergestellt, dass sich sowohl die Form des Gerätekörpers als auch der Bereich des Schaftes außerhalb des Gerätekörpers nicht verformen. Es ist allerdings auch denkbar, dass die Formteile wenigstens bereichsweise eine andere Form aufweisen als die genannten Komponenten, sodass sich die Form der Komponenten lokal ändern lässt. Im weiteren Schritt werden offene Enden des mindestens einen Gerätekörpers und/oder des mindestens einen rohrartigen Schaftes durch Dichtmittel verschlossen. Bei diesen Dichtmitteln kann es sich um Ventile handeln, Schraub- oder Druckverschlüsse oder sonstige Stopfen. Über mindestens ein Ventil, welches vorzugsweise in einem der Dichtmittel integriert ist, wird sodann ein Druck, insbesondere ein hydraulischer oder ein pneumatischer Druck, aufgebaut. Durch diesen Druck wird der mindestens eine Bereich des mindestens einen rohrartigen Schaftes, der sich innerhalb des Aufnahmeraums des Gerätekörpers befindet, einer Innenkontur des Gerätekörpers angepasst. Durch diese Aufweitung des Abschnitts entsteht die formschlüssige Verbindung zwischen dem rohrartigen Schaft und dem Gerätekörper. Diese durch das IHU-Verfahren hergestellte Verbindung genügt den höchsten Qualitätsanforderungen an die chirurgischen Handgeräte. Durch anschließende Druckreduzierung sowie Öffnen der Form ist die Verbindung zwischen dem rohrartigen Schaft und dem Körper wenigstens nahezu fertiggestellt.

Ein weiteres bevorzugtes Ausführungsbeispiel der Erfindung sieht es vor, dass die Innenkontur des mindestens einen Gerätekörpers mindestens einen ringartigen Hinterschnitt und/oder mindestens einen Vorsprung aufweist, gegen den der mindestens eine rohrartige Schaft formschlüssig gedrückt wird. Dieser Hinterschnitt bzw. Vorsprung oder die Aussparung verleiht der Verbindung zwischen dem Schaft und dem Körper eine zusätzliche Festigkeit. Insbesondere bezüglich Kräften, die axial auf den Schaft wirken, können somit aufgefangen werden und zwar ohne dass dabei die Verbindung zwischen den Komponenten zerstört wird. Es ist außerdem denkbar, dass die Innenkontur eine Vielzahl von Hinterschnitten bzw. Vorsprüngen aufweist.

Vorzugsweise kann es weiter vorgesehen sein, dass der mindestens eine rohrartige Schaft mit einem vordefinierten Druck beaufschlagt wird oder der Druck erhöht wird, bis eine ausreichend feste formschlüssige Verbindung hergestellt ist. Der auf die Innenwandung des Schaftes wirkende Druck kann sowohl über das Ventil als auch an einem externen Druckgenerator, welcher über einen entsprechenden Zulauf mit dem Schaft verbunden ist, geregelt werden. Der Druck, mit dem der Schaft beaufschlagt wird, kann in Abhängigkeit geregelt werden vom Material der Komponenten sowie von der Form und Dimension des Schaftes. Um einen genügend hohen Druck für die formschlüssige Verbindung aufzubauen, können entweder vorgewählte Druckwerte eingestellt werden oder die Änderung des Druckes als Indiz für die Aufweitung des Schaftes herangezogen werden.

Ein chirurgisches Handgerät zur Lösung der eingangs genannten Aufgabe wird durch die Merkmale des Anspruchs 8 beschrieben. Demnach ist es vorgesehen, dass das chirurgische Handgerät, bei dem es sich um ein Endoskop, ein Resektoskop, ein Zystoskop oder dergleichen handelt, nach einem Verfahren gemäß Anspruch 1 hergestellt werden. Das chirurgische Handgerät besteht im Wesentlichen aus mindestens einem Gerätekörper bzw. einem Hauptkörper, einem Anschlusskörper oder einem Konus des Gerätekörpers oder einer Optikplatte oder einer anderen Komponente. Des Weiteren besteht das Handgerät aus mindestens einem rohrartigen Schaft, einem Rohr oder einem Außenrohr oder einem Innenrohr. Der mindestens eine Körper weist einen Aufnahmeraum bzw. einen Konus auf, der in seinem Querschnitt gegenüber dem Querschnitt des rohrartigen Schaftes geringfügig größer ist. Ansonsten korrespondiert der Querschnitt des Schaftes mit der Innenkontur des Aufnahmeraums.

Sowohl der Gerätekörper als auch der Schaft können aus einem Metall, wie beispielsweise Edelstahl, Titan oder dergleichen, hergestellt sein. Diese Materialien lassen sich mittels des IHU-Verfahrens besonders gut verformen.

Des Weiteren kann es erfindungsgemäß vorgesehen sein, dass eine Innenkontur des mindestens einen Gerätekörpers mindestens einen ringartigen Hinterschnitt und/oder mindestens einen Vorsprung aufweist, gegen den der mindestens eine rohrartige Schaft formschlüssig gedrückt wird. Durch diese Hinterschnitte bzw. Vorsprünge kann der Formschluss zwischen den genannten Komponenten verbessert werden.

Neben den hier genannten chirurgischen Geräten ist es denkbar, dass auch andere Geräte durch die beschriebenen Maßnahmen hergestellt werden bzw. die genannten Merkmale aufweisen.

Ein bevorzugtes Ausführungsbeispiel der Erfindung wird nachfolgend anhand der Zeichnung näher beschrieben. In dieser zeigen:
- Fig. 1: eine schematische Darstellung eines Resektoskops,
- Fig. 2: eine schematische Teildarstellung eines Hauptkörpers und eines Schafts, und
- Fig. 3: eine schematische Teildarstellung eines Hauptkörpers und eines Schafts.

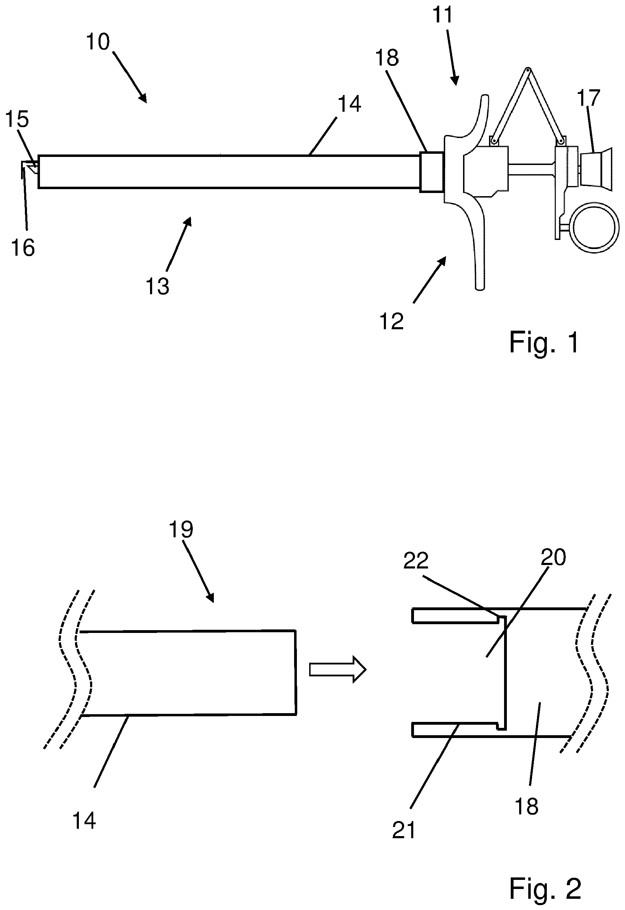

In der Fig. 1 ist beispielhaft für ein chirurgisches Handgerät ein Resektoskop 10 dargestellt. Dieses Resektoskop 10 besteht im Wesentlichen aus einem Transporteur 11, einer Griffeinheit 12 und einem Schaft 13. Es sei ausdrücklich darauf hingewiesen, dass es sich bei dem chirurgischen Handgerät auch um ein anderes Gerät als das hier dargestellte Resektoskop 10 handeln kann. Das in der Fig. 1 dargestellte Resektoskop 10 dient lediglich dazu, die Erfindung zu illustrieren. Wie bereits ausgeführt, lassen sich die erfindungsgemäßen Maßnahmen und Merkmale auch auf andere chirurgische Geräte übertragen sowie an mehreren Komponenten, bzw. Körpern und Schäften, gleichzeitig oder nacheinander durchführen.

Bei dem hier dargestellten Ausführungsbeispiel eines chirurgischen Handgeräts setzt sich der Schaft 13 zusammen aus einem äußeren Schaftrohr 14, einer Optik 15 und einem Elektrodeninstrument 16. Die Optik 15 besteht aus einem langen Rohr, in welchem Linsen oder Glasfasern angeordnet sein können, um durch ein proximal an dem Schaft 13 angeordnetes Okular 17 einen Bereich am distalen Ende des Schaftes 13 beobachten zu können. Für eine detailliertere Beschreibung des hier dargestellten Resektoskops 10 wird auf den bekannten Stand der Technik verwiesen.

Wie üblich weist auch das in der Fig. 1 dargestellte chirurgische Handinstrument bzw. das Resektoskop 10 einen Gerätekörper 18 auf. Dieser Gerätekörper 18, auch als Hauptkörper oder Anschlusskörper bezeichnet, stellt einen wesentlichen Teil des Gerätes dar. Beispielsweise kann an jedem Hauptkörper 18 die Griffeinheit 12 angeordnet sein oder weitere Komponenten für die Handhabung oder Steuerung des Instrumentes. Im Falle des hier dargestellten Resektoskops 10 wird durch den Gerätekörper 18 unter anderem die Optik 15 geführt.

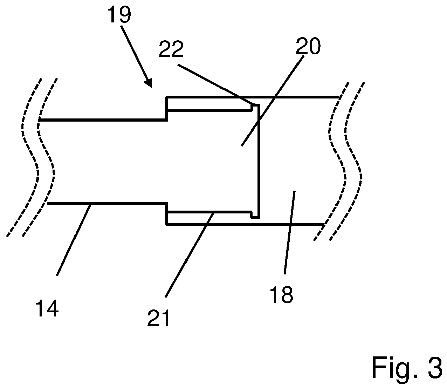

An dem Gerätekörper 18 ist der rohrartige Schaft 13 fest verbunden. In dem Schaft 13 werden in Abhängigkeit von der Art des chirurgischen Handgerätes verschiedene Instrumente geführt, wodurch diese vor äußeren Einflüssen geschützt werden. Gemäß der vorliegenden Erfindung ist ein proximaler Endbereich 19 des Schaftes 13 in einem Aufnahmeraum 20 des Gehäusekörpers 18 formschlüssig verbunden. Dazu wird zunächst der Endbereich 19 des Schaftes 13 in den Aufnahmeraum 20 des Körpers 18 geführt (Fig. 2). Durch Beaufschlagung des Schaftes 13 mit einem hydraulischen Druck passt sich der distale Endbereich des Schaftes 13 an eine innere Kontur 21 des Gerätekörpers 18 an (Fig. 3). Die innere Kontur 21 des Gerätekörpers 18 kann dabei einen ringartigen Hinterschnitt 22 aufweisen, in den sich der Schaft 13 fügt. Durch dieses Aufweiten des proximalen Endbereichs 19 des Schaftes 13 wird die formschlüssige Verbindung hergestellt. Dies ist stark schematisiert in der Fig. 3 dargestellt.

Bei dem hier beschriebenen Verfahren zur Herstellung des chirurgischen Handgeräts ist ein Außendurchmesser des Schafts 13 nur unwesentlich kleiner als die Innenmaße des Aufnahmeraums 20 des Gerätekörpers 18. Wäre der Unterschiede zwischen diesen Maßen zu groß, würde das Umformen des Schafts 13 bzw. die Anpassung des Umfangs des Schafts 13 an die Innenkontur 21 zu einem unbefriedigenden Ergebnis führen. Zum einen könnte kein oder ein ungenügender Formschluss hergestellt werden und zum anderen würde sich die Form des Schafts 13 bzw. des Endbereichs 19 des Schafts 13 wahrscheinlich derartig verändern, dass es zu einer Materialschwäche käme. Sofern der Unterschied zwischen den genannten Maßen nur gering ist, passt sich der äußere Umfang des Schafts 13 optimal der inneren Kontur 21 des Gerätekörpers 18 an, und zwar ohne dass die Form bzw. die Materialbeschaffenheit des Schafts 13 durch den Umformungsprozess in Mitleidenschaft gezogen werden würde. Darüber hinaus wird durch diese verhältnismäßig geringe Umformung ein geringerer Kraftaufwand benötigt.

Damit die auf die Wandung des Schafts 13 einwirkende Kraft eben nur den Endbereich 19 des Schafts 13 umformt, nicht aber den gesamten Schaft sowie den Hauptkörper, ist es erfindungsgemäß vorgesehen, dass vor der Druckbeaufschlagung sowohl der Gerätekörper 18 als auch der Schaft 13, der bereits im Aufnahmeraum 20 positioniert ist, von einer hier nicht dargestellten Form umschlossen wird. Diese Form kann idealerweise aus zwei Hälften bestehen. Es ist jedoch auch denkbar, dass die Form, insbesondere für komplexe Formen des Gerätekörpers 18, aus mehreren Elementen zusammensetzbar ist. Die Form, insbesondere die zusammengesetzte Form, ist derart ausgebildet, dass sie den auf den Schaft 13 wirkenden Druck standhält und somit eine Umformung des Schafts 13 und des Körpers 18 verhindert.

Es ist allerdings auch denkbar, dass die Form bzw. die Formelemente Hinterschnitte oder dergleichen aufweisen, so dass, insbesondere der Schaft 13, die Komponenten des Handgeräts zusätzliche Umformungen durch die Druckbeaufschlagung erhalten. Durch diese zusätzliche Umformung können die Komponenten für spezielle Anwendungen bzw. Anforderungen vorbereitet werden.

Die Beaufschlagung des hydraulischen oder pneumatischen Drucks erfolgt durch einen entsprechenden, nicht dargestellten Generator. Dieser Generator ist über eine Leitung und einen Anschluss mit dem Schaft 13 und/oder dem Gerätekörper 18 verbunden. Für eine geregelte bzw. gesteuerte Druckerzeugung kann zwischen der Leitung und den genannten Komponenten wenigstens ein Ventil angeordnet sein. Die Kopplung der Leitung bzw. des Ventils mit dem Schaft 13 oder dem Gerätekörper 18 kann über einen speziellen Anschluss bzw. Flansch oder dergleichen erfolgen. Nach Beendigung des Umformprozesses wird der Druck reduziert und die Verbindung entkoppelt. Die Komponenten sind danach lediglich von dem gegebenenfalls verwendeten Fluid zu reinigen. Somit ist die Umformung des Schaftabschnitts bzw. die formschlüssige Verbindung zwischen dem Schaft 13 und dem Gerätekörper 18 reduziert auf wenige, einfach durchzuführende Schritte.

Durch das Einführen des Schaftendes 19 in den Aufnahmeraum 20, das Fixieren der Komponenten in der Form sowie durch die Druckbeaufschlagung ist das Verfahren besonders einfach sowie reproduzierbar. Wenigstens einige dieser Schritte lassen sich vollautomatisch durchführen, so dass dieses erfindungsgemäße Verfahren auch zu Kosteneinsparung führt.

Der Querschnitt des Schafts 13 ist bei diesem Verfahren nicht auf einen Kreis bzw. Ring beschränkt. Vielmehr kann der Querschnitt des Schafts 13 sowie der Querschnitt des Aufnahmeraums 20 auch eine andere Form aufweisen, wie beispielsweise eine Ellipse oder beliebiges ein Oval oder dergleichen. Auch polygonale Querschnittsformen sind denkbar. Durch eine Kombination mehrerer, insbesondere unterschiedlich geformter Hinterschnitte 22 sowie vorzugsweise Vorsprünge an der Innenkontur 21 des Gerätekörpers 18 lässt sich ein besonders zuverlässiger sowie stabiler Formschluss herstellen.

Neben den in den Fig. 2 und 3 dargestellten Ausführungsformen, insbesondere die Form des Aufnahmeraums 20, der Innenkontur sowie der Hinterschnitte 22, sind auch andere Formen als die hier dargestellten vorgesehen.

### Bezugszeichenliste

- 10: Resektoskop
- 11: Transporteur
- 12: Griffeinheit
- 13: Schaft
- 14: äußeres Schaftrohr
- 15: Optik
- 16: Elektrodeninstrument
- 17: Okular
- 18: Gerätekörper
- 19: Endbereich des Schaftes
- 20: Aufnahmeraum
- 21: innere Kontur
- 22: Hinterschnitt

## Patentansprüche

1. Verfahren zur Herstellung eines chirurgischen Handgeräts, insbesondere eines Endoskops, eines Resektoskops (10), eines Zystoskops oder dergleichen, mit mindestens einem rohrartigen Schaft (13) und mindestens einem Gerätekörper (18), **dadurch gekennzeichnet, dass** mindestens ein Gerätekörper (18) und mindestens ein rohrartiger Schaft (13) durch Innen-Hochdruck-Umformung miteinander formschlüssig verbunden werden.

2. Verfahren zur Herstellung eines chirurgischen Handgeräts nach Anspruch 1, **dadurch gekennzeichnet, dass** es sich bei dem mindestens einem Gerätekörper (18) um einen Hauptkörper oder einen Anschlusskörper oder einen Konus des Gerätekörpers (18) oder eine weitere Komponente des Handgeräts, insbesondere eine Optikplatte, handelt.

3. Verfahren zur Herstellung eines chirurgischen Handgeräts nach einem der Ansprüche 1 oder 2, **dadurch gekennzeichnet, dass** es sich bei dem mindestens einem rohrartigen Schaft (13) um einen Schaft, ein Rohr oder ein Außenrohr oder ein Innenrohr handelt.

4. Verfahren zur Herstellung eines chirurgischen Handgeräts nach einem der vorangegangenen Ansprüche, **dadurch gekennzeichnet, dass**
a) mindestens ein Abschnitt, vorzugsweise ein Endabschnitt (19) und/oder ein mittlerer Abschnitt, des mindestens einen rohrartigen Schaftes (13) in einen Aufnahmeraum (20) des mindestens einen Gerätekörpers (18) geführt wird;
b) der mindestens eine Gerätekörper (18) und der mindestens eine rohrartige Schaft (13) zusammen wenigstens bereichsweise in mindestens einer Form, die vorzugsweise aus zwei Formhälften zusammengesetzt wird, fixiert werden;
c) offene Enden des mindestens einen Gerätekörpers (18) und/oder des mindestens einen rohrartigen Schafts (13) durch Dichtmittel verschlossen werden;
d) über mindestens ein Ventil, das vorzugsweise in einem Dichtmittel integriert ist, ein hydraulischer Druck in dem mindestens einen Schaft (13) aufgebaut wird;
d) der mindestens eine Abschnitt (19) des mindestens einen rohrartigen Schaftes (13), der sich innerhalb des Aufnahmeraums (20) des mindestens einen Gerätekörpers (18) befindet, wird durch die Druckbeaufschlagung an eine Innenkontur (21) des mindestens einen Gerätekörpers (18) gedrückt, wodurch die formschlüssige Verbindung zwischen dem mindestens einen rohrartigen Schaft (13) und dem mindestens einen Gerätekörper (18) erzeugt wird.

5. Verfahren zur Herstellung eines chirurgischen Handgeräts nach einem der vorangegangenen Ansprüche, **dadurch gekennzeichnet, dass** die Innenkontur (21) des mindestens einen Gerätekörpers (18) mindestens einen ringartigen Hinterschnitt (22) und/oder mindestens einen Vorsprung aufweist, gegen den der mindestens eine rohrartige Schaft (13) formschlüssig gedrückt wird.

6. Verfahren zur Herstellung eines chirurgischen Handgeräts nach einem der vorangegangenen Ansprüche, **dadurch gekennzeichnet, dass** der mindestens eine rohrartige Schaft (13) mit einem vordefinierten Druck beaufschlagt wird oder der Druck solange erhöht wird, bis eine ausreichend feste formschlüssige Verbindung hergestellt ist.

7. Verfahren zur Herstellung eines chirurgischen Handgeräts nach einem der vorangegangenen Ansprüche, **dadurch gekennzeichnet, dass** zwei Gerätekörper (18) oder mehr gleichzeitig oder nacheinander mit einem, zwei oder mehr rohrartigen Schäften (13) durch Innen-Hochdruck-Umformung formschlüssig verbunden werden oder dass zwei oder mehrere rohrartige Schäfte gleichzeitig oder nacheinander miteinander durch Innen-Hochdruck-Umformung formschlüssig verbunden werden.

8. Chirurgischen Handgeräts, insbesondere eines Endoskops, eines Resektoskops (10), eines Zystoskops oder dergleichen, mit mindestens einem rohrartigen Schaft (13) und mindestens einem Gerätekörper (18), hergestellt nach einem Verfahren gemäß Anspruch 1.

9. Chirurgisches Handgerät nach Anspruch 8, **dadurch gekennzeichnet, dass** es sich bei dem mindestens einen Gerätekörper (18) um einen Hauptkörper oder einen Anschlusskörper oder einen Konus des Gerätekörpers (18) oder eine weitere Komponente des Handgeräts, insbesondere eine Optikplatte, handelt.

10. Chirurgisches Handgerät nach Anspruch 8 oder 9, **dadurch gekennzeichnet, dass** es sich bei dem mindestens einen rohrartigen Schaft (13) um einen Schaft, ein Rohr oder ein Außenrohr oder ein Innenrohr handelt.

11. Chirurgisches Handgerät nach einem der Ansprüche 8 bis 10, **dadurch gekennzeichnet, dass** eine Innenkontur (21) des mindestens einen Gerätekörpers (18) mindestens einen ringartigen Hinterschnitt (22) und/oder mindestens einen Vorsprung aufweist, gegen den der mindestens eine rohrartige Schaft (13) formschlüssig gedrückt wird.
